# NEUE EUROPÄISCHE PATENTSCHRIFT

(11) **EP 2 160 166 B2**
(45) Veröffentlichungstag und Bekanntmachung des Hinweises auf die Entscheidung über den Einspruch: **28.09.2022**
(45) Hinweis auf die Patenterteilung: 01.01.2014
(21) Anmeldenummer: 08759161.6
(22) Anmeldetag: 11.06.2008
(51) Int. Cl.: A61F 13/02

(54) **WUNDVERBAND**
WOUND DRESSING
PANSEMENT

(30) Priorität: 27.06.2007 DE 102007029796
(43) Veröffentlichungstag der Anmeldung: 10.03.2010
(73) Patentinhaber: Paul Hartmann AG, 89522 Heidenheim (DE)
(72) Erfinder: ECKSTEIN, Axel, 89522 Heidenheim (DE); GOERL, Rudolf, 63785 Obernburg (DE)
(74) Vertreter: DREISS Patentanwälte PartG mbB
(86) Internationale Anmeldenummer: PCT/EP2008/004643
(87) Internationale Veröffentlichungsnummer: WO 2009/000416

(56) Entgegenhaltungen:
- EP-A- 0 594 034
- EP-A- 1 462 075
- EP-A1- 0 114 481
- EP-A1- 1 462 075
- WO-A-91/01122
- WO-A-2004/112852
- WO-A1-91/01122
- WO-A1-91/01706
- WO-A1-2004/112852
- WO-A2-03/099344
- WO-A2-2004/103415
- DE-A1- 19 916 523
- GB-A- 2 425 487
- GB-A- 2 425 487
- US-A- 4 655 768
- US-B1- 6 191 336
- K.L. Ulman, X. Thomas:"Silicone pressure sensitive adhesives for healthcare applications", Advances in pressure sensitive technology-2 (1995),S. 133-137

## Beschreibung

Die Erfindung betrifft einen Wundverband umfassend ein aktiviertes oder vor der Wundbehandlung zu aktivierendes Wundkissen zur Wundbehandlung im feuchten Milieu mit einem flächigen Trägermaterial, das das Wundkissen zumindest bereichsweise überragt, wobei das Trägermaterial auf der wundzugewandten Seite zumindest bereichsweise mit einer Klebeschicht versehen ist zum Fixieren des Wundverbandes an einem Träger.

Derartige Wundverbände sind im Stand der Technik bekannt. So beschreibt beispielsweise die WO 91/01122 ein Verbandmaterial umfassend eine wasserundurchlässige mikroporöse Kunststoffträgerschicht und einem darauf aufgebrachten flüssigkeitsaufnehmenden Wundkissen, wobei das flüssigkeitsaufnehmende Wundkissen mit Wasser oder einer wässrigen Lösung getränkt ist. Des Weiteren ist eine Klebeschicht vorgesehen, um das Verbandmaterial an einem Träger zu fixieren.

Des Weiteren ist beispielsweise aus der EP 1 462 075 ein Wundverband bekannt mit einer Befestigung und einem Abdeckteil sowie einem Wundkissen, wobei der Befestigungsteil zum einen an einer Haut eines Trägers klebend befestigbar ist und zum anderen einen Klettverschluss aufweist, mit dem er mit dem Abdeckteil zusammenwirkt.

Die DE 199 16 523 A1 offenbart ein Pflaster mit einer Trägerschicht und einem Wundkissen, das auf der körperzugewandten Seite der Trägerschicht vorgesehen ist, sowie einer Stützschicht, die auf der wundabgewandten Seite der Trägerschicht, die auf Folienbasis gebildet ist, aufgebracht ist. Zur Verbindung zwischen Trägerschicht und Stützschicht ist eine Verklebung vorgesehen, wobei nach Aufbringen des Pflasters die Stützschicht von der Trägerschicht abgezogen werden kann, wobei eine Einzelumverpackung des Pflasters vorgesehen ist, und eine Hälfte der Einzelumverpackung die Stützschicht bildet.

Eine Wundkompresse zur Behandlung im feuchten Milieu offenbart die EP 0 594 034 B1 mit einem Quellkörper, welcher in einer mindestens teilweise flüssigkeitsdurchlässigen Hülle angeordnet ist, zur Speicherung und kontinuierlichen Abgabe einer Flüssigkeit, wobei ein Superabsorbierer im Quellkörper vorgesehen ist, der im gequollenen Zustand eine gelartige Konsistenz aufweist, und wobei die Hülle wenigstens teilweise aus einem textilen Material besteht, das weich und plastisch verformbar, jedoch nicht elastisch ist.

Ausgehend von diesem Stand der Technik stellt sich die Erfindung die Aufgabe, einen weiter verbesserten Wundverband bereitzustellen, bei dem die Fixierung und Applikation des Wundverbandes vereinfacht ist und der darüber hinaus den Vorteil besitzt, sterilisierbar zu sein.

Die Erfindung löst diese Aufgabe, durch einen Wundverband mit den Merkmalen des Anspruchs 1. Es können jedoch Zusätze und Additive unter 50 Gew.-%, insbesondere unter 30 Gew.-% und insbesondere unter 10 Gew.-% enthalten sein.

Als druckempfindliche Silikonhaftkleber können insbesondere solche Silikonhaftkleber verwendet werden, die in K.L. Ulman, X. Thomas "Silicone pressure sensitive adhesives for healthcare applications", Advances in pressure sensitive technology-2 (1995), S. 133-157 beschrieben sind.

Durch die Verwendung von Silikonklebern anstelle der sonst üblichen im Verbandmaterialbereich eingesetzten Klebstoffen, wie Naturkautschuk, Acrylatklebern, sowie sonstigen Hotmeltklebern besteht der Vorteil, dass sie zum einen ein Fixieren der Wundauflage erlauben, sowohl bei trockenen als auch bei Verwendung von nassen Wundkissen, d. h., in den Fällen, in denen das Wundkissen mit Flüssigkeit durchtränkt und somit aktiviert ist, wobei unter Durchtränken verstanden werden soll, dass das flüssigkeitsaufnehmende Material möglichst viel Flüssigkeit aufgenommen hat und die druckempfindlichen Silikonhaftkleber neben ihrer hautfreundlichen Charakteristik darüber hinaus eine Hitzesterilisation erlauben, ohne dass der Kleber kollabiert und somit seine Klebekräfte einbüßt. Es ist damit der besondere Verdienst der vorliegenden Erfindung, festgestellt zu haben, dass bei Verwendung von druckempfindlichen Silikonhaftklebern nicht nur Klebeeigenschaften sowohl auf feuchten als auch nassen Oberflächen bereitgestellt werden können, sondern auch eine Hitzesterilisation, beispielsweise in Form einer Ethyloxidsterilisation, möglich ist, so dass ein derartiger Wundverband auch im sterilen Bereich eingesetzt werden kann, wobei ein Einsatz besonders dort vorteilhaft ist, wo in der Praxis mehr als "zwei Hände" notwendig sind, um eine Applikation sicherzustellen, ohne dass die Wundauflage gegebenenfalls in einen unsterilen Bereich gelangt und somit ihre Brauchbarkeit verliert. Die verbesserte Applikation wird durch die Verwendung eines Trägermaterials erreicht, das derart klebend beschichtet ist, dass es an einem Träger, also einem Patienten, wobei als Patient sowohl Menschen als auch Tiere in Betracht kommen, klebend festgelegt werden kann.

Dabei wird unter Aktivieren das Tränken oder Befeuchten des Wundkissens verstanden, das die Flüssigkeit zwischenspeichert, um sie dann nach Applikation an einem Träger insbesondere verzögert freizusetzen.

Dabei kann vorgesehen sein, dass als Trägermaterial ein solches Material eingesetzt wird, das im Bereich von 60 bis 150 °C hitzebeständig bzw. hitzestabil ist. Dabei kann es sich insbesondere um ein textiles Material und vorzugsweise um ein Nonwoven handeln, wobei Polyethylenvliese als bevorzugte Materialien eingesetzt werden.

Dabei kann generell vorgesehen sein, dass die Klebeschicht auf der wundzugewandten Seite vollflächig oder auch lediglich abschnittsweise, insbesondere in Mustern, aufgebracht ist. Durch einen Auftrag in Mustern kann die Wasserdampfdurchlässigkeit des Trägermaterials bei einem applizierten erfindungsgemäßen Wundverband verbessert werden. Damit kann der Tatsache, dass die Wasserdampfdurchlässigkeit eines Wundverbandes im Bereich eines Kleberauftrags stets herabgesetzt ist und die druckempfindlichen Silikonhaftkleber, die zwar hautverträglich sind, jedoch nur eine begrenzte Wasserdampfdurchlässigkeit aufweisen, entgegengewirkt werden.

Insbesondere kann vorteilhaft vorgesehen sein, dass die Klebeschicht eine Dicke von 40 bis 500 µm, vorzugsweise 150 bis 450 µm, und besonders bevorzugt 250 bis 400 µm besitzt. Besonders bevorzugte kann eine Dicke der Klebeschicht von 350 bis 400 µm vorgesehen sein. Das Flächengewicht, also die Auftragsmenge, ist dabei vorzugsweise < 375 g/m².

Des Weiteren kann vorgesehen sein, dass die Klebkraft eines entsprechenden druckempfindlichen Silikonhaftklebers ca. 0,8 N/25mm, insbesondere 0,85 bis 0,95 N/25 mm und besonders bevorzugt 0,92 N/25 mm entspricht, wobei die Klebkraft nach dem Deutschen Arzneimittelbuch DAB 10 von 1996 mit einem Abzugswinkel von 180° gegenüber rostfreiem Stahl gemessen wurde.

Darüber hinaus kann die Klebschicht eine Wasserdampfdurchlässigkeit (moisture vapor transition rate MVTR (upright)) von 800 bis 920 g/m2/24h, insbesondere 850 bis 900 g/m²/24h aufweisen. Dieser sogenannte upright-Wert wurde nach DIN EN 13726-2 (2002) gemessen. Darüber hinaus wurde eine Wasserdampfdurchlässigkeit (MVTR (inverted)) von 2500 bis 3000 g/m²/24h nach DIN EN 13726-2 (2002) bestimmt. Hierbei handelt es sich um eine Wasserdampfdurchlässigkeit bei direktem Kontakt mit der Klebeschicht.
Bei dem Wundkissen handelt es sich um ein nicht wund verklebendes Wundkissen, das eine Hülle aus einem nicht-wundverklebenden Polymermaterial umfasst. Diese Hülle besteht aus einem nicht-wund verklebenden Gestrick, Gewirk oder Gewebe, das weiterhin bevorzugt aus einem hydrophoben Fasermaterial besteht. Insbesondere kann die Hülle ein Gestrick, Gewirk oder Gewebe aus einem hydrophoben Polyethylen-, Polypropylen-, Polyester-oder Viskose-Polymermaterial sein. Durch die Ausgestaltung als Gestrick, Gewirk oder Gewebe kann die Hülle in einer oder mehreren Richtungen gedehnt oder verformt werden, ohne dass sie sich von selbst wieder zusammenzieht oder ausrichtet. Die Oberfläche einer derartigen Hülle gleicht sich zudem passgenau an die Oberfläche der zu behandelnden Haut oder Wunde an.

Weiterhin überdeckt das Trägermaterial das Wundkissen nicht allseitig. Zwar ist generell vorgesehen, dass das Trägermaterial zumindest bereichsweise über das Wundkissen hinaussteht, um eine Fixierung an einem Träger zu gewährleisten. Es ist jedoch grundsätzlich auch denkbar, dass es sich bei dem Trägermaterial lediglich um ein streifenförmiges oder ringförmiges Material mit einer Mittelaussparung, in die zumindest bereichsweise das Wundkissen aufgenommen ist, handelt. Eine Vielzahl von anderen Formen ist denkbar, sofern das Wundkissen am Wundverband festgelegt ist und über das Trägermaterial eine Fixierung an einem Träger erfolgen kann. Dabei ist es möglich, das Trägermaterial als wie auch immer gestaltete Netz- oder Lochstruktur bereitzustellen, wobei wichtig ist, dass das Trägermaterial das Wundkissen weit genug überspannt, so dass ein sicheres Fixieren des Wundkissens auf einer Wunde sichergestellt werden kann.

Es ist vorgesehen, dass das Wundkissen über einen Rahmen, der teilweise das Wundkissen überdeckt, wobei der Rahmen unmittelbar oder mittelbar am Trägermaterial festgelegt ist, gehalten ist. Das Wundkissen kann dann klebend oder "schwimmend" zwischen Trägermaterial und Rahmen gelagertsein, wo-beials Wundkontakt bereich der Bereich der Aussparung im Rahmen dient.

Darüber hinaus kann vorgesehen sein, dass die wundzugewandte Seite des Klebers mit einer im Bereich von 60 bis 150 °C hitzestabilen Schutzschicht versehen ist.

Bei der Schutzschicht kann es sich insbesondere um eine Folie, insbesondere eine nicht quellbare Polymerfolie, handeln. Alternativ kann die Schutzschicht auch aus einem Silikonpapier bestehen. Die Abdeckung mit einer solchen Schutzschicht, die auch als Liner bezeichnet werden kann, besitzt den Vorteil, dass kein Anhaften des Wundverbandes, beispielsweise an einer Umverpackung, erfolgt und so ein leichteres Entnehmen des Wundverbandes aus der Umverpackung gewährleistet ist. Darüber hinaus kann vorgesehen sein, dass durch die Schutzschicht nicht nur die wundzugewandte Klebeschicht, die zur Fixierung an einem Träger dient, überdeckt ist, sondern zugleich das Wundkissen geschützt ist, so dass dieses erst kurz vor der Applikation an einem Träger freigelegt wird.

Es kann des Weiteren vorgesehen sein, dass die Schutzschicht durch eine Umverpackung des Wundverbandes bereitgestellt ist. Auf diese Weise kann eine weitere Materialschicht eingespart werden, und darüber hinaus wird eine Fixierung des Wundverbandes in der Umverpackung, die insbesondere als mit einer Folie verschlossene Tiefziehpackung oder als flacher Folienbeutel vorgesehen sein kann, erreicht. Der Wundverband kann dabei im nassen oder auch im trockenen Zustand, also vor der Aktivierung, im Inneren einer solchen Umverpackung gelagert sein.

Es kann vorgesehen sein, dass das Wundkissen einen Quellkörper insbesondere mit einem superabsorbierenden Material enthält. Dabei kann insbesondere ein Aufbau eines Quellkörpers sowie eines Wundverbandes gemäß der EP 594 034 A1, der als Produkt "TenderWet ^{®}" der Firma Paul Hartmann AG, Heidenheim, erhältlich ist, als Wundkissen beim erfindungsgemäßen Verfahren eingesetzt werden.

Dabei kann vorgesehen sein, dass der Quellkörper oder das Wundkissen aus einem Sandwich aus zwei Polypropylengestricken und einem dazwischen angeordneten Faser/SAP-Gemisch gebildet sind.

Die Erfindung soll im Folgenden anhand eines Beispiels zunächst näher erläutert werden.

Als Wundkissen wurde eine Kompresse der Marke TenderWet^{®} der Firma Paul Hartmann AG, Heidenheim, verwendet, wobei das so gebildete Wundkissen eine erste Abstandsschicht, darüber liegend eine erste Verteilerschicht, darüber liegend ein absorbierendes Kissen aus einem im airlaid-Verfahren hergestellten Faser/SAP-Gemisch sowie darüber angeordnet eine zweite Verteiler- und eine zweite Abstandsschicht aufweist. Die beiden Abstandsschichten sind entlang ihrer Ränder miteinander verbunden, so dass diese eine geschlossene Hülle für die absorbierende und für die zwei Verteilerschichten bilden.

Die Abstandsschichten sind jeweils aus einem hydrophoben Gestrick aus Polypropylen gefertigt, das im entspannten Zustand eine Dicke von 0,8 mm, gemessen bei einem Prüfdruck von 0,5 kPa aufweist. Durch die gute Permeabilität der Abstandsschichten wird die Flüssigkeit sehr rasch durch die Abstandsschicht hindurch zur Wunde oder von der Wunde her in das Depot abgeleitet. Das eigentliche Wundkissen weist dabei sowohl eine feuchtigkeitsabgebende als auch eine absorbierende Wirkung auf. Dabei nimmt die Abstandsschicht kaum Feuchtigkeit auf. Die beiden Verteilerschichten sind aus einem aus 100% Zellulosefasern gefertigten Tissue mit einem Flächengewicht von 18 g/m² hergestellt. Das Innere des Wundkissens besitzt ein Flächengewicht von 340 g/m² und besteht aus einem luftgelegten (airlaid) 1:1-Gemisch aus Fasern und superabsorbierendem Natriumpolyacrylat (Favor^{®} Z 3034 der Firma Degussa). Das Natriumpolyacrylat liegt in Partikelform vor und weist einen Partikelanteil von ca. 99% auf, der eine Partikelgröße von < 0,85 mm besitzt. Ca. 80% der Partikel weisen eine Größe von 0,3 bis 0,85 mm auf, gemessen nach EDANA 420.2-02. Das Fasermaterial ist ein Gemisch aus 94 Gew.-% 100%-igen hydrophilen Zellulosefasern und 6 Gew.-% 100%-igen Polypropylenfasern. Das Wundkissen ist mittels einer wässrigen Lösung aktiviert, wobei die wässrige Lösung aus einer isotonischen Elektrolytlösung enthaltend Wasser, Magnesiumchlorid, Kalziumchlorid und Kaliumchlorid besteht.

Als Trägermaterial dient ein Nonwoven (Bioflex RX1247 PLT der Firma Scapa), das mit einem druckempfindlichen Silikonhaftkleber als Klebeschicht beschichtet wurde und an das durch die druckempfindliche Silikonhaftkleberbeschichtung das Wundkissen angeheftet wurde. Durch die vollflächige Abdeckung des Wundkissens mit dem druckempfindlichen Silikonhaftkleber kann das Wundkissen weniger leicht austrocknen, wodurch längere Verweilzeiten auf einer Wunde realisiert werden können.

Der so hergestellte Wundverband wurde in einer als Umverpackung dienenden Tiefziehpackung fixiert, indem die über das Wundkissen hinausstehenden Bereich des Trägermaterials mit dem Inneren der Umverpackung klebend verbunden wurden.

Es wurde anschließend bei 53 ± 2°C eine Ethylenoxid-(EO)-Sterilisation im Unterdruckverfahren durchgeführt. Dabei wurde ein Dampfpartialdruck von 105 mbar sowie eine EO-Gaskonzentration von 800 g/m³ Ethylenoxid verwendet. Das Sterilisationsgas war ein Gemisch aus EO/CO₂ (90/10). Nach 180 min Einwirkzeit wurde das Produkt in der Kammer mit fünf Lüftungszyklen gespült und danach der Desorption für 24 h (ca. 25° C) zugeführt.

Alternativ zu dem hier beschriebenen Ethylenoxidsterilisations-Verfahren kann eine Wasserdampfsterilisation mit Gegendruck verwendet werden. Hierbei können Temperaturen von 90 - 130° C verwendet werden.

Eine Kollabierung des Klebstoffs fand nicht statt. Eine nachfolgende Aktivierung des Wundkissens mit 8 bis 10 ml Ringerlösung führte zu einer Separierung des Trägermaterials vom Wundkissen.

Weiterhin soll die Erfindung im Folgenden anhand einer Zeichnung näher erläutert werden. Dabei zeigen:
- Figuren 1 bis 4: verschiedene Ausführungsformen in einer Draufsicht; und die
- Figuren 5 bis 8: verschiedene Ausgestaltungen im Schnitt.

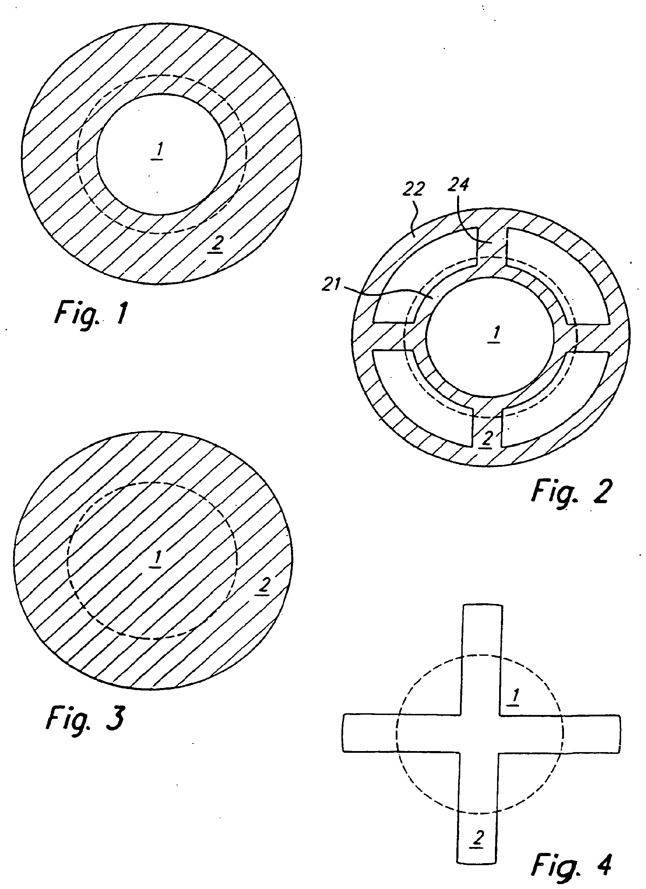

Figur 1 zeigt eine erste Ausgestaltung umfassend ein Wundkissen 1 sowie ein Trägermaterial 2. Das flächige Trägermaterial 2 ist hierbei ringförmig gestaltet und weist in der Mitte eine Aussparung auf. Die Aussparung ist dabei im Durchmesser geringfügig kleiner als das Wundkissen, so dass über den gesamten Umfang des Wundkissens dieses durch das Trägermaterial 2 überdeckt ist und am Trägermaterial klebend fixiert werden kann. Dazu ist auf das Trägermaterial 2 eine Klebeschicht (nicht dargestellt) vollflächig oder partiell aufgetragen. Durch die nicht vorgesehene Abdeckung im Bereich des Wundkissens 1 wird im Bereich des Wundkissens 1 die Wasserdampfdurchlässigkeit verbessert.

Eine alternative Ausgestaltung zeigt Figur 2, wobei hier ebenfalls eine ringförmige Grundform des Trägermaterials 2 vorgesehen ist, jedoch aus dem Ring vier Segmente ausgenommen wurden, so dass sich eine rippenartige Verbindung zwischen einem inneren und einem äußeren Ring des Trägermaterials 2 ergibt, wobei der innere Ring mit 21 und der äußere Ring mit 22 sowie die dazwischen liegenden Rippen mit 24 bezeichnet sind.

Hierdurch kann bei gleichermaßen guter Fixierung des Wundverbandes an einem Träger über das Trägermaterial 2 die Wasserdampfdurchlässigkeit auch im Bereich des Trägermaterials 2 weiter erhöht werden, wobei jedoch die Applikationsfähigkeit verändert ist, da das Trägermaterial instabiler ist.

Figur 3 zeigt dahingegen eine nicht erfindungsgemäße Ausgestaltung, bei der das Trägermaterial 2 vollflächig ausgebildet ist und eine Kreisform besitzt. Es überspannt somit das gesamte Wundkissen 1.

Eine weitere alternative Ausgestaltung zeigt Figur 4, wobei hier ebenfalls ein rundes Wundkissen 1 vorgesehen ist, sowie ein kreuzförmig vorgesehenes Trägermaterial 2, das in zwei senkrecht aufeinander stehenden Richtungen in einer Ebene das Wundkissen 1 überspannt und dieses so an einem Träger fixierbar macht.

Bei allen vorstehenden Ausgestaltungen trägt dabei die dem Wundkissen 1 zugewandte Seite des Trägermaterials 2 eine Klebeschicht umfassend einen druckempfindlichen Silikonhaftkleber.

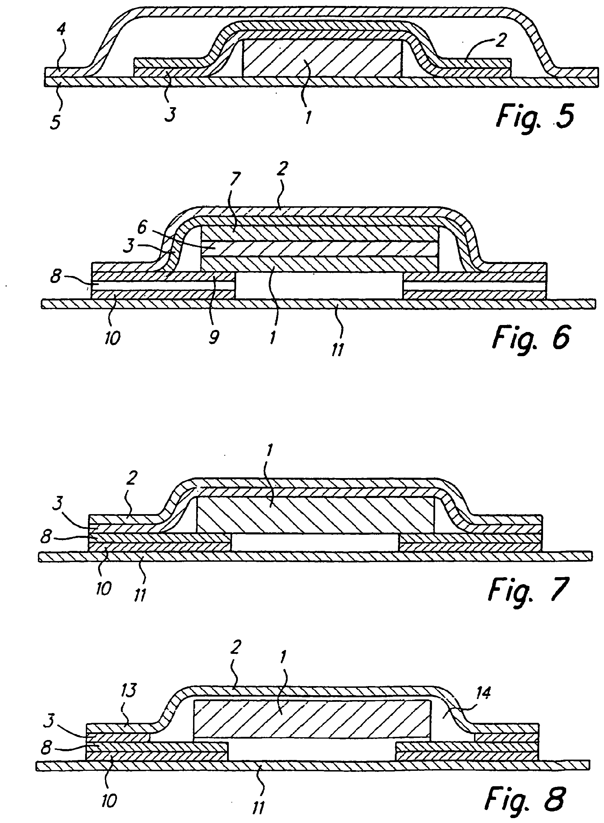

Figur 5 zeigt nun eine nicht erfindungsgemäße Ausgestaltung eines Wundverbandes im Schnitt, wobei ein Wundkissen 1 vorgesehen ist, sowie ein Trägermaterial 2. Das Wundkissen ist an dem Trägermaterial mittels eines druckempfindlichen Silikonhaftklebers festgelegt, wobei der druckempfindliche Silikonhaftkleber in Form einer Klebeschicht 3 auf das Trägermaterial 2 aufgebracht ist. Der Kleberauftrag kann dabei sowohl partiell und insbesondere in Mustern als auch vollflächig erfolgen. Die so gebildete Wundauflage bzw. der so gebildete Wundverband wird dann vorzugsweise in einer Umverpackung konfektioniert, wobei diese aus einem Tiefziehbehältnis mit einem oberen Tiefziehelement 4 und einem unteren Tiefziehelement 5 besteht.

Das untere Tiefziehelement 5 dient dabei hier zugleich als Schutzschicht für die Klebeschicht 3 des Trägermaterials 2, so dass der Wundverband an der Umverpackung und hier insbesondere am unteren Teil 5 der Tiefziehpackung klebend festgelegt ist.

Eine erfindungsgemäße Ausgestaltung zeigt Figur 6, wobei hier gleiche Elemente mit gleichen Bezugszeichen versehen sind. Das Wundkissen 1 ist gemäß Figur 6 durch ein Sandwich gebildet, wobei eine Lage eines Airlaid-Faser/SAP-Quellkörpers 6 zwischen zwei Polypropylengestricke, die hydrophob ausgebildet und mit dem Bezugszeichen 7 versehen ist, laminiert wurde. Dabei sorgen die Polypropylengestricke für die gute Weiterleitung und Abgabe von Flüssigkeit sowohl in den Quellkörper 6 als auch aus dem Quellkörper 6 hinaus. Das Trägermaterial 2 ist wiederum mit einem druckempfindlichen Silikonhaftkleber als Klebeschicht 3 beschichtet, wobei darüber hinaus ein Rahmen 8 vorgesehen ist, der aus dem gleichen Material wie das Trägermaterial 2 gebildet ist, und wobei der Rahmen 8 mittels Klebeschichten 9 und 10, die ebenfalls druckempfindliche Silikonhaftkleber umfassen, an einem Träger sowie Wundkissen festlegbar ist. Die druckempfindlichen Silkonhaftkleber sind dabei hitzebeständig bei einer Sterilisation und auch im nassen Milieu klebend, zum einen am Wundkissen 1 und zum anderen an einer Schutzschicht 11, die entweder durch eine Umverpackung oder einem sonstigen Material, beispielsweise einem Silikonpapier gebildet sein kann, und auch an einem Patienten während der Anwendung. Durch die Vorsehung eines Rahmens wird erreicht, dass das eigentliche Wundkissen 1 von einer Wunde beabstandet ist und so ein Verkleben mit einer Wunde und damit eine Zerstörung der sich neu bildenden Hautzellen weiter verhindert werden kann. Eine weitergehende Verminderung des Risikos erfolgt durch die Verwendung eines feuchten bzw. aktivierbaren Wundkissens, da diese eine verringerte Neigung zum Anhaften an eine Wunde aufweisen.

Dabei ist der Rahmen 8 beidseitig mit einer Klebeschicht 9, 10 beschichtet.

Eine ähnliche Ausgestaltung zeigt Figur 7, wobei hier das Trägermaterial 2 vollflächig mit einer Klebeschicht 3 versehen ist, wobei die Klebeschicht 3 im Bereich des Wundkissens 1 dieses an dem Trägermaterial 2 fixiert. Des Weiteren ist an den nicht durch das Wundkissen 1 überdeckten Bereichen des Trägermaterials 2 ein Rahmen 8 vorgesehen, der über die Klebeschicht 3, die auf dem Trägermaterial 2 aufgebracht ist, angehaftet wurde. Der Rahmen 8 ist bei diesem Ausführungsbeispiel nicht klebend mit dem Wundkissen 1 verbunden. Der Rahmen 8 trägt nun auf seiner einer Hautoberfläche eines Trägers zugewandten Seite wiederum eine Klebebeschichtung 10, die auf Basis von druckempfindlichen Silikonhaftklebern gebildet ist, und wird mit dieser Klebebeschichtung 10 entweder auf einem Schutzmaterial 11 oder nach Applikation auf der Haut eines Trägers fixiert.

Figur 8 zeigt eine weitere Ausgestaltung, bei der das Trägermaterial 2 im Bereich des Wundkissens 1 keine Klebeschicht 3 aufweist, sondern lediglich in seinen über das Wundkissen 1 hinausgehenden Bereichen 13. In diesen Bereichen 13 ist das Trägermaterial 2 mit einem Rahmen 8 verbunden, wobei der Rahmen sich in den Bereich des Wundkissens 1 hineinerstreckt, und dieses ein Stück weit überlappt, ohne mit diesem klebend verbunden zu sein. Das Wundkissen 1 ist bei dieser Ausgestaltung "schwimmend" zwischen dem Rahmen 8 und dem Trägermaterial 2 gehalten und kann sich im Bereich des so gebildeten Raumes 14 frei zwischen Trägermaterial 2 und Rahmen 8 bewegen. Der Rahmen 8 ist dann wiederum wie in Figur 7 auf seiner einer Hautoberfläche zugewandten Seite mit einer Klebeschicht 10 umfassend und insbesondere bestehend aus druckempfindlichen Silikonhaftklebern beschichtet, die eine Sterilisierbarkeit sowie eine Klebebeständigkeit auf feuchten und nassen Oberflächen gewährleistet. Vor der Applikation und während der Lagerung ist der Wundverband mit der Klebeschicht 10 auf einer Schutzschicht 11 festgelegt.

Auf die vorstehend beschriebene Weise kann besonders einfach ein sterilisierbarer Wundverband, der einfach zu applizieren ist, bereitgestellt werden, wobei es sich um einen Wundverband mit aktiviertem oder aktivierbarem Wundkissen handelt, das im nassen bzw. feuchten Zustand auf eine Hautoberfläche aufgebracht.

## Patentansprüche

1. Wundverband umfassend ein aktiviertes oder vor der Wundbehandlung zu aktivierendes Wundkissen (1) zur Wundbehandlung im feuchten Milieu mit einem flächigen Trägermaterial (2), das das Wundkissen (1) zumindest bereichsweise überragt, wobei das Trägermaterial (2) auf einer wundzugewandten Seite zumindest bereichsweise mit einer Kleberschicht (3) versehen ist zum Fixieren des Wundverbandes an einem Träger, wobei die Klebeschicht (3) ein oder mehrere druckempfindliche Silikonhaftkleber als Kleber umfasst und insbesondere aus druckempfindlichen Silikonhaftklebern besteht und wobei auf der wundzugewandten Seite des Trägermaterials (2) ein Rahmen (8) aufgebracht ist und das Trägermaterial (2) über den Rahmen (8) an einem Träger festlegbar ist, **dadurch gekennzeichnet, dass** das Trägermaterial (2) nicht vollflächig ausgebildet ist und das Wundkissen ein nichtwundverklebendes Wundkissen ist, das eine Hülle aus einem nicht-wundverklebenden Polymermaterial umfasst, und diese Hülle aus einem nicht-wundverklebenden Gestrick, Gewirk oder Gewebe besteht, die in eine oder mehrere Richtungen gedehnt oder verformt werden kann, ohne dass sie sich von selbst wieder zusammenzieht oder ausrichtet, und wobei der Rahmen (8) zumindest in Teilbereichen die wundzugewandte Seite des Wundkissens (1) überdeckt.

2. Wundverband nach Anspruch 1, **dadurch gekennzeichnet, dass** das Trägermaterial (2) aus einem in Bereich von 60 bis 150° C hitzestabilen Material, vorzugsweise einem textilen Material und vorzugsweise einem Nonwoven, insbesondere einem PE-Vlies besteht.

3. Wundverband nach einem der vorangehenden Ansprüche, **dadurch gekennzeichnet, dass** das Wundkissen (1) über einen Bereich der Klebeschicht (3) an dem Trägermaterial (2) festgelegt ist.

4. Wundverband nach einem der vorangehenden Ansprüche, **dadurch gekennzeichnet, dass** das Wundkissen (1) einen Quellkörper (6), insbesondere mit einem superabsorbierenden Material enthält.

5. Wundverband nach Anspruch 1 oder 2, **dadurch gekennzeichnet, dass** die wundzugewandte Seite der Kleberschicht (3) mit einer Schutzschicht (11) insbesondere aus einem im Bereich von 60 bis 150°C hitzestabilen Material, insbesondere einer Folie, insbesondere einer nichtquellbaren Polymerfolie oder einem Silikonpapier abgedeckt ist.

6. Wundverband nach einem der vorangehenden Ansprüche, **dadurch gekennzeichnet, dass** die Schutzschicht (11) durch eine Umverpackung des Wundverbandes bereitgestellt ist.

7. Wundverband nach einem der vorangehenden Ansprüche, **dadurch gekennzeichnet, dass** die Klebeschicht (3) eine Dicke von 40 bis 500 µm, vorzugsweise 40 bis 350 µm und besonders bevorzugt 40 bis 200 µm aufweist.

8. Wundverband nach einem der vorangehenden Ansprüche, **dadurch gekennzeichnet, dass** das Wundkissen (1) ein Sandwich aus zwei Polypropylen-Gestricken (7) und einem dazwischen liegenden Quellkörper (6) aus Faser/SAP-Gemisch umfasst.

## Claims

1. Wound dressing, comprising a wound pad (1) that is activated or to be activated before the wound is treated, for wound treatment in a moist environment, having a flat substrate material (2) which protrudes beyond the wound pad (1) at least in regions, the substrate material (2) being provided, on a wound-facing side, at least in regions, with an adhesive layer (3) for fixing the wound dressing to a substrate, the adhesive layer (3) comprising one or more pressure-sensitive silicone adhesives as the adhesive and in particular consisting of pressure-sensitive silicone adhesives, and a frame (8) being attached to the wound-facing side of the substrate material (2) and the substrate material (2) being able to be fixed on a substrate via the frame (8), **characterized in that** the substrate material (2) is not formed over the entire surface and the wound pad is a non-wound-adhering wound pad which has a sheath made of a non-wound-adhering polymer material, and this sheath consists of a non-wound-adhering weft-knitted, warp-knitted or woven fabric which can be expanded or deformed in one or more directions without contracting or aligning itself again, and the frame (8) covering the wound-facing side of the wound pad (1) at least in portions.

2. Wound dressing according to claim 1, **characterized in that** the substrate material (2) consists of a material that is heat-stable in the range from 60 to 150°C, preferably a textile material and preferably a nonwoven, in particular a PE nonwoven.

3. Wound dressing according to either of the preceding claims, **characterized in that** the wound pad (1) is fixed to the substrate material (2) via a region of the adhesive layer (3).

4. Wound dressing according to any of the preceding claims, **characterized in that** the wound pad (1) contains a swelling body (6), in particular having a superabsorbent material.

5. Wound dressing according to claim 1 or claim 2, **characterized in that** the wound-facing side of the adhesive layer (3) is covered with a protective layer (11) in particular made of a material that is heat-stable in the range from 60 to 150°C, in particular a film, in particular a non-swellable polymer film or a silicone paper.

6. Wound dressing according to any of the preceding claims, **characterized in that** the protective layer (11) is provided by an outer packaging of the wound dressing.

7. Wound dressing according to any of the preceding claims, **characterized in that** the adhesive layer (3) has a thickness of 40 to 500 µm, preferably 40 to 350 µm and particularly preferably 40 to 200 µm.

8. Wound dressing according to any of the preceding claims, **characterized in that** the wound pad (1) comprises a sandwich consisting of two polypropylene weft-knitted fabrics (7) and a swelling body (6) consisting of a fiber-SAP mixture located therebetween.

## Revendications

1. Pansement comprenant un coussinet (1) activé ou à activer avant le traitement des plaies pour le traitement des plaies en milieu humide avec un matériau de support (2) plat, qui fait saillie du coussinet (1) au moins par endroits, dans lequel le matériau de support (2) est pourvu sur une face tournée vers la plaie au moins par endroits d'une couche adhésive (3) pour la fixation du pansement sur un porteur, dans lequel la couche adhésive (3) comprend un ou plusieurs auto-adhésifs en silicone sensibles à la pression comme adhésifs et en particulier est constituée d'auto-adhésifs en silicone sensibles à la pression et dans lequel un cadre (8) est appliqué sur la face du matériau de support (2) tournée vers la plaie et le matériau de support (2) peut être immobilisé sur un porteur par l'intermédiaire du cadre (8), **caractérisé en ce que** le matériau de support (2) n'est pas réalisé sur toute la surface et le coussinet est un coussinet n'adhérant pas à la plaie, qui comprend une enveloppe composée d'un matériau polymère n'adhérant pas à la plaie, et cette enveloppe est constituée d'un tricot, article à mailles ou tissu n'adhérant pas à la plaie, qui peut être étiré ou déformé dans une ou plusieurs directions, sans qu'il se contracte à nouveau ou s'oriente de lui-même, et dans lequel le cadre (8) recouvre au moins dans des zones partielles la face du coussinet (1) tournée vers la plaie.

2. Pansement selon la revendication 1, **caractérisé en ce que** le matériau de support (2) est constitué d'un matériau stable à la chaleur dans la plage de 60 à 150 °C, de préférence d'un matériau textile et de préférence d'un non-tissé, en particulier d'un non-tissé en PE.

3. Pansement selon l'une quelconque des revendications précédentes, **caractérisé en ce que** le coussinet (1) est immobilisé sur le matériau de support (2) par l'intermédiaire d'une zone de la couche adhésive (3).

4. Pansement selon l'une quelconque des revendications précédentes, **caractérisé en ce que** le coussinet (1) contient un corps gonflant (6), en particulier avec un matériau superabsorbant.

5. Pansement selon la revendication 1 ou 2, **caractérisé en ce que** la face de la couche adhésive (3) tournée vers la plaie est recouverte avec une couche protectrice (11) en particulier composée d'un matériau stable à la chaleur dans la plage de 60 à 150 °C, en particulier d'un film, en particulier d'un film polymère ne pouvant gonfler ou d'un papier siliconé.

6. Pansement selon l'une quelconque des revendications précédentes, **caractérisé en ce que** la couche protectrice (11) est fournie par un suremballage du pansement.

7. Pansement selon l'une quelconque des revendications précédentes, **caractérisé en ce que** la couche adhésive (3) présente une épaisseur de 40 à 500 µm, de préférence 40 à 350 µm et de manière particulièrement préférée 40 à 200 µm.

8. Pansement selon l'une quelconque des revendications précédentes, **caractérisé en ce que** le coussinet (1) comprend un sandwich composé de deux tricots de polypropylène (7) et d'un corps gonflant (6) situé entre ceux-ci composé d'un mélange de fibres et de PSA.
